# EUROPEAN PATENT APPLICATION

(11) **EP 3 288 009 A1**
(43) Date of publication of application: **28.02.2018**
(21) Application number: 17186550.4
(22) Date of filing: 17.08.2017
(51) Int. Cl.: G09B 23/28, G09B 23/30

(54) **LOW-RESISTANCE ANATOMICAL TISSUE MODEL**

(30) Priority: 23.08.2016 US 201662378254 P
(71) Applicant: Virtamed AG, 8952 Schlieren (CH)
(72) Inventor: O'BRIEN, Carolyn, 8952 Schlieren (CH); WETTENGEL, Kelly, 8952 Schlieren (CH)
(74) Representative: Leman Consulting S.A.

(57) **Abstract**

Anatomy mannequins, models and medical simulation systems described herein may facilitate various medical procedure training by rendering a realistic passive haptic touch when the end user manipulates the anatomy model with a handheld tool. An anatomy tissue model may comprise at least one fabric layer arranged over at least one foam layer to replicate the low-friction, elasticity and hardness of a living body anatomy tissue, such as the vagina wall, without requiring the use of lubricant in the medical simulation training procedure. The anatomy tissue model may be attached as an insert to a body structure model. A diversity of anatomy tissue models may be adapted with different fabric layer and/or foam layer designs, each design corresponding to a different haptic touch, so they can be interchanged in a medical training simulator system to realistically replicate different medical training scenarios.

## Description

### FIELD

The present invention relates to anatomy models in general, and more specifically to anatomical tissue models for medical simulation purposes.

### BACKGROUND

### Medical simulation

Medical imaging has become more and more used for both diagnostic/examination and therapeutic purposes in a number of medical applications, such as endoscopy for surgery, or ultrasound imaging for various gynecology and/or obstetrics applications. These new techniques may require dedicated training for physicians and surgeons to master the indirect hand-eye coordination required by the imaging system as well as the manipulation of the imaging tools, such as the endoscope or the ultrasound probe, in addition to the conventional medical instruments and procedures for a diversity of patient anatomies as may be encountered in medical practice.

Computerized medical procedure training simulators may enable the physicians and trainees to develop and improve their practice in a virtual reality environment before actually practicing in the operation room.

Advanced medical procedure simulators may be based on a virtual reality ("VR") and/or a mixed or augmented reality ("AR") simulation apparatus by which the physician can experiment a medical procedure scenario. The VR/AR system may compute and display a visual VR/AR model of anatomical structures in accordance with physician gestures and actions to provide various feedback, such as visual feedback. In a VR system, an entire image may be simulated for display to a user, and in an AR system, a simulated image may be overlaid or otherwise incorporated with an actual image for display to a user. Various patient models with different pathologies can be selected.

Therefore, natural variations as encountered over the years by practicing doctors can be simulated for a user over a compressed period of time for training purposes. The medical simulation procedure can be recorded and rehearsed for evaluation purpose. The VR/AR simulation system can also compute and provide various metrics and statistics.

VR/AR simulation systems such as the one described in US patent 8992230 include a human anatomy model in real size. The VR/AR simulation system may further comprise a medical instrument to be handheld by the user to more realistically simulate the medical procedure. A passive feedback VR/AR simulation system such as for instance the one described in US patent 8992230 may also be used with a diversity of medical procedure training scenarios, some of which may possibly result in a mismatch between an anatomy model surface as touched by the trainee and a virtual environment surface as computed by the VR/AR simulation system and rendered on the screen. In order to further improve the passive haptic experience and increase the realism in such medical training scenarios, the VR/AR simulation system may be further adapted with space warping methods and systems as described in US patent 9330502.

### Passive haptics

For passive haptic VR/AR medical simulators, maximizing the realism of interacting with the physical anatomy model (mannequin) with medical tools or instruments directly adapted from the real medical practice may further improve the learning experience. In ultrasound procedures, the user manipulates the an ultrasound transducer by pressing it on the anatomy tissue and positioning and orienting it to optimize the ultrasound imaging capture. In real medical practice, an ultrasound gel is used to facilitate the ultrasound propagation as well as the patient comfort during manipulation of the ultrasound transducer in contact with the patient tissue. However, in a simulator, in general, it is not desirable to use any liquid or gel in a training setup due to the overhead required to clean it. The lack of lubricant when operating the tool against the anatomy model surfaces may cause a less realistic haptic experience when moving the simulator probe over the anatomy model surface compared to moving a real ultrasound probe over a body surface such as the belly or the vagina walls. Moreover, friction of the rigid plastic simulator probe tip over the anatomy model surface significantly increases when the user has to push the probe to compress the anatomy tissue and get a better ultrasound image. This friction is much lower in a real procedure where ultrasound gel or jelly is used to improve the ultrasound capture.

A system to improve the haptic feedback in a passive VR/AR simulator is described in US patent application 62/242,322 where the tool is adapted to interact with low friction with a surface of the anatomy model, such as the belly in simulating an abdominal ultrasound procedure. However in the case of internal examination of body anatomy cavities, such as for instance transvaginal ultrasound, upper gastrointestinal endoscopy, or colonoscopy, the tool is surrounded by the anatomy tissue walls, which may complicate its mechanical adaptation to provide a realistic low-friction touch to the simulator tool user.

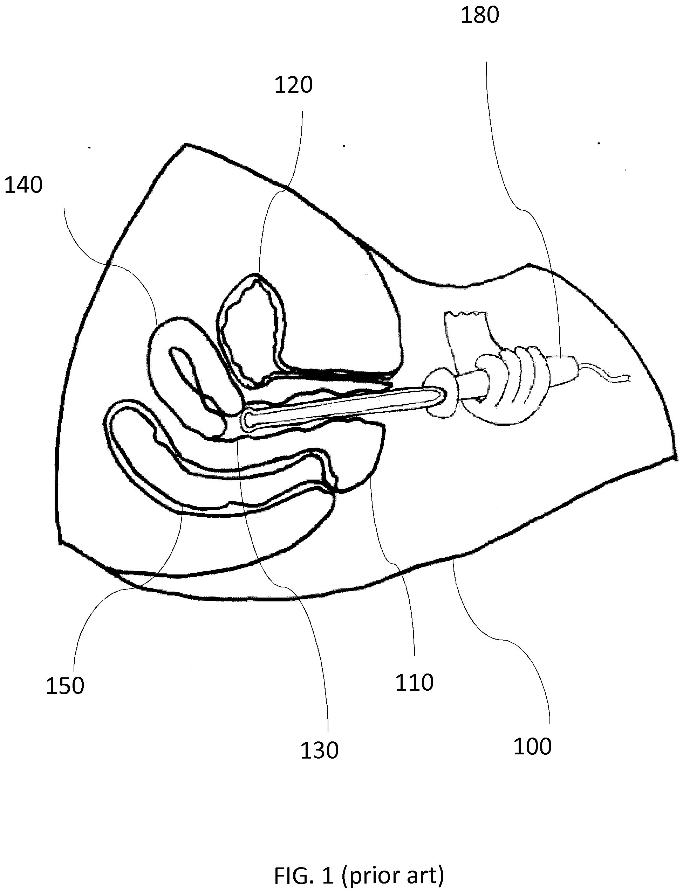

In a transvaginal ultrasound examination procedure as represented for example by FIG.1, the user manipulates a transvaginal ultrasound transducer tool 180 by introducing it into the vagina 130 and positioning and orienting it along the walls of vagina 130 to optimize the ultrasound imaging capture. In real medical procedures, lubricant is used to facilitate the ultrasound propagation as well as the patient comfort during manipulation of the ultrasound transducer in contact with the vagina wall. From a passive haptic perspective, one major challenge is the lack of realism when manipulating the transvaginal plastic probe simulator in vagina anatomy models which use rubber for all elements of the anatomy. More generally, rubber has a number of advantages for cost-effective medical simulation anatomy models, such as being robust, cheap and easy to maintain, but it is a high-friction material and therefore it requires the use of lubricant to provide a realistic haptic feedback when manipulating a tool 180 such as a transvaginal probe, an endoscope, a catheter or a speculum in contact with the walls inside the anatomy model cavity 130. However, lubricants are difficult to clean, may cause long-term degradation, and are not well accepted by medical simulation users. Lubricants, when they dry out, attract dirt and dust, and due to the anatomical form of some models such as conventional vagina anatomy models, special tools or large amounts of time are required to fully remove the lubricant out from the anatomy model rubber surface. Solutions based on condoms would still require lubricants.

Solutions based on polytetraflouroethylene (PTFE) and other plastics material offer the low coefficient of friction that does not require the use of lubricant, but they lack the other material properties that are needed for a realistic passive feedback in manipulating the tool against the anatomy simulator walls, such as the right combination of elasticity and hardness.

There is therefore a need for a low-resistance anatomical tissue model that facilitates realistic passive feedback when manipulating tools in contact with the anatomy model for a diversity of anatomy simulation applications without requiring an expensive material design and/or cumbersome lubricant application and cleaning procedures.

### BRIEF SUMMARY

It is an object of the present disclosure to provide an anatomical tissue model for simulating the interaction of a tool with a living body tissue, comprising a fabric layer arranged over a foam layer to replicate the low-friction, elasticity and hardness of a the living body tissue when touching the tissue with the tool.

The fabric layer may comprise at least one of PCM (phase change material), nylon or another synthetic polyamide, polyester, cotton, rayon, dyneema, Kevlar, Nomex, Lycra, spandex, acrylic fibres, or a combination thereof, with at least 5% of synthetic elastic fibre such as elastane, Lycra, or Spandex. The fabric layer may weight of at least 80g per square meter and at most 330g per square meter.

The foam layer may be a polyether foam, with a load deflection of at least 1.5kPa and at most 19kPa and
a weight of at least 15kg and at most 110kg per cube meter.

The fabric layer and the foam layer may be tethered by at least one tethering spot by at least one screw or a glue spot.

It is another object of the present disclosure to provide a mannequin comprising at least one body structure, which may be made of plastic, silicon, rubber or polyurethane, and at least one anatomical tissue model system comprising a fabric layer arranged over a foam layer to replicate the low-friction, elasticity and hardness of a the living body tissue when touching the tissue with the tool. At least one of the fabric layer or the foam layer of the anatomic tissue model system may be tethered to the mannequin structure. At least one part of the fabric layer or at least one part of the foam layer of the anatomic tissue model system may be glued or screwed to the mannequin structure.

It is a further object of the present disclosure to provide an anatomical tissue model insert for a mannequin, the mannequin comprising a cavity structure, the anatomical tissue model insert being adapted to fast mount in the cavity structure, the anatomical tissue model insert comprising at least one fabric layer arranged over at least one foam layer to replicate the low-friction, elasticity and hardness of a living body anatomical tissue. The fabric layer and the foam layer may be tethered by at least one tethering spot. The fabric layer and the foam layer may be screwed or glued together.

The anatomy model insert may further comprise an external structure as an injection molded part, made of foam, plastic, or other suitable materials, to facilitate the insertion of the anatomy model into the mannequin with a sliding fit. The fabric layer and/or the foam layer may be firmly attached to the anatomy model external structure with various tethering techniques, such as glueing or screwing.

### BRIEF DESCRIPTION OF THE DRAWINGS

FIG. 1 illustrates a medical procedure of the prior art as may be replicated by a medical training simulator, comprising a mannequin structure, an anatomy model such as the vagina model, and a tool such as an endoscope which is inserted and manipulated by the user into the anatomy model.
FIG. 2 provides a partial sectional view of a low-resistance anatomy tissue model insert according to a possible embodiment.
FIG. 3 shows a partial view of a mannequin adapted with a low-resistance anatomy tissue model insert for upper gastrointestinal endoscopy simulation.

### DETAILED DESCRIPTION

Some embodiments of low-friction realistic anatomy models will now be described in more detail with reference to an exemplary transvaginal ultrasound simulation system, as illustrated on FIG.1.

FIG.1 shows a partial view of a prior art pelvic mannequin 100 comprising pelvic anatomy structures such as an pelvis model 110, a bladder model 120, a vagina model 130, an uterine model 140, and a lower bowel model 150. The clinical female pelvic model (CPFT) Mk 3 product by Limbs&Things (product information: https://www.limbsandthings.com/global/our-products/details/clinical-female-pelvic-trainer-mk-3-standard) may be used, but other pelvic models may apply as well. The anatomy model parts 110, 120, 130, 140, 150 may be made of plastic, silicon, rubber, polyurethane, or any other suitable material. In general, the anatomy model parts is made of a flexible material, such as flexible plastic, so that it can deform under pressure.

Different vagina 130 and/or uterine 140 models may be interchanged to simulate different medical practices, for instance respectively corresponding to a normal nulliparous patient, a multiparous patient with a retroverted uterine, or a 10-12 weeks pregnant patient. The transvaginal ultrasound system also comprises standard tools such as an ultrasound probe replicate 180 and a speculum (not illustrated) to facilitate the insertion of the ultrasound probe into the vagina. The user interacts with the simulator system mannequin 100 by manipulating the ultrasound probe 180 in the vagina model 130.

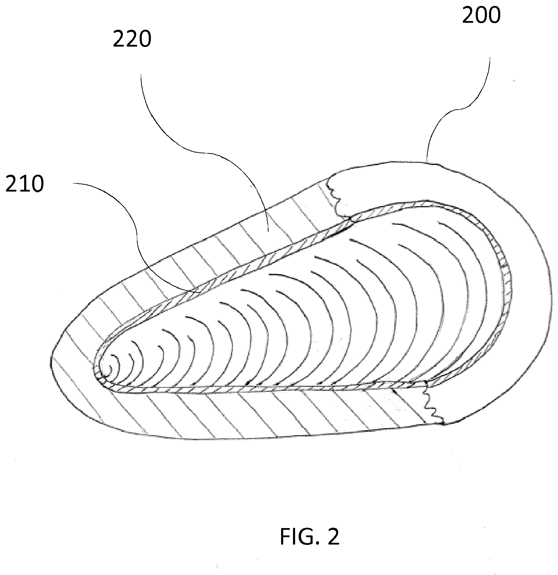

FIG. 2 shows a partial sectional view of a vagina model insert 200 in accordance with a possible embodiment of the present disclosure, where the anatomical model 200 may comprise a fabric layer 210 arranged over a foam layer 220 to replicate the low-friction, elasticity and hardeness of the anatomical model tissue walls in real world examination of living patients with ultrasound probe lubricant.

The fabric layer 210 may be made of a low cost textile material, such as PCM (phase change material), nylon or another synthetic polyamide, polyester, cotton, rayon, dyneema, Kevlar, Nomex, Lycra, spandex, acrylic fibres, or a combination thereof. In a possible embodiment, the fabric layer 210 may be made of cotton combined with at least 5% of synthetic elastic fibre such as elastane, Lycra^{™} or Spandex^{™} material, with a weight in the range of 80g to 330g per square meter. It was shown experimentally that a fabric layer with at most 330g per square meter provides an elasticity and low-friction haptic touch similar to real practice in standard OBGYN transvaginal examination.

The foam layer 220 may provide additional hardness to further increase the realism. In a possible embodiment, a foam layer made of polyether with a load deflection of 5.1kPa and a weight of 25kg per cube meter provides a hardness haptic touch similar to real practice in standard OBGYN transvaginal examination, when used in combination with a fabric layer 210 manufactured with at most 330g per square meter, but other embodiments are also possible. In general, a load deflection in the range of 1.5kPa to 19kPa and a density in the range of 15kg to 110kg per cube meter for the foam layer 220 may be used to simulate with a cost effective design various hardness haptic touches similar to the interaction with anatomy tissues as may be encountered in medical practice.

Depending on the needs of the medical simulation and the actual simulation system, the fabric layer and the foam layer may be either employed together in a loose way and separately attached to the medical simulation system mannequin 100 with various tethering techniques, or they may be tethered to each other by at least one tethering spot to form an anatomy model insert, for instance as a replacement for the prior art vagina model 130, to be inserted into a medical simulation system mannequin 100. In a possible embodiment, the fabric layer and the foam layer may be glued together at least one tethering spot. In another possible embodiment, they may be glued together by large contact surfaces. In another possible embodiment, they may be screwed together. Other embodiments are also possible, for instance a combination of gluing and screwing, or any other tethering means, that may be either chemical, mechanical, or magnetic, as will be apparent to those skilled in the art of mechanical engineering.

Similarly, the anatomy model insert may also be further attached to the medical simulation system mannequin with various tethering techniques. In a possible embodiment, with reference to FIG.1 as an illustrative example, the fabric layer may be attached to the vulva of the pelvic model 110 on the one hand and to the cervix of the uterine model 140 on the other hand, while the foam layer is attached to the cervix of the uterine model 140 only, but other embodiments are also possible.

In a possible embodiment, the anatomy model insert may be attached to the medical simulation system by a fast mechanical mount system, so that it can be easily plugged in and out, removed and interchanged with other anatomy model inserts, for instance by clipping it on and off the mannequin structure. To facilitate the fast mount, in a possible embodiment, the anatomy model insert may further comprise an external structure as an injection molded part, made of foam, plastic, or other suitable materials, to facilitate the insertion of the anatomy model into the mannequin with a sliding fit. The fabric layer and/or the foam layer may then be firmly attached to the anatomy model external structure with various tethering techniques, such as glueing or screwing, so that they can be easily plugged in and out the mannequin as part of the sliding insert.

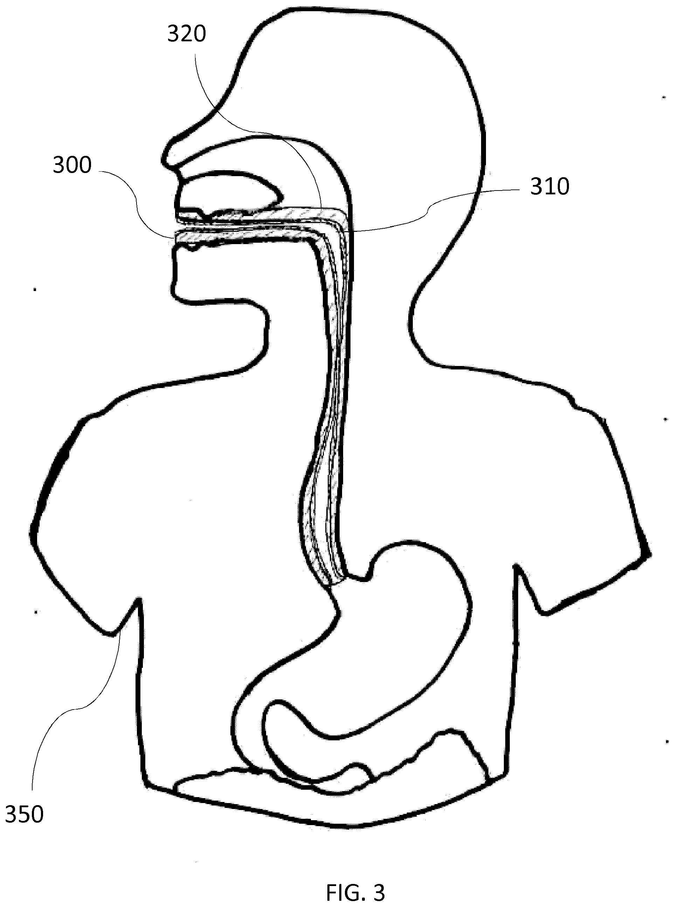

While the vagina anatomy model for transvaginal ultrasound practice simulation has been detailed so far herein as an example for a better illustration of the disclosure, the proposed models and systems can be generalized to other anatomy models and medical simulations, such as the esophagus for upper gastrointestinal endoscopy or the lower bowel for colonoscopy. FIG. 3 shows a partial view of an exemplary embodiment of an upper gastrointestinal tube model insert 300 attached to an upper torso and head mannequin 350 that may be employed by a gastrointestinal endoscopy training simulator. The upper gastrointestinal tube model insert 300 of FIG.3 comprises a fabric layer 310 and a foam layer 320 arranged to replicate the haptic touch of an upper gastrointestinal tube when the user inserts a tool such as an endoscope (not represented) into the upper gastrointestinal tube model 300.

The actual material composition and/or density parameters of the fabric layer and/or the foam layer may be adapted to match the actual touch of different organ tissues with a tool, in accordance with medical practice experience, for different applications.

Furthermore, the actual material composition and density of the fabric layer and/or the foam layer may also be adjusted to provide interchangeable anatomy tissue models corresponding to different various medical scenarios and/or pathologies in a passive haptic medical training simulator.

Different fabric layer and/or foam layer designs may for instance simulate an increased hardness due to uterine and baby volume at different pregnancy stages, or due to the presence of cancer tumors. A fabric layer design with a lower friction may also replicate the presence of overproduction of mucus as may be caused by certain medical conditions such as tissue inflammation, cystic fibrosis, etc.

Different combinations of the material composition and/or the density parameters may also be arranged for different parts of the fabric layer and/or the foam layer in an interchangeable anatomy model insert to replicate the heterogeneity of the anatomy tissue walls as may be met in certain specific medical practice scenarios. A fabric layer with a lower weight may be netting and thus replicate rougher, bumpier tissues, possibly in certain disease conditions. Higher weight fabric layers may replicate stiffer tissues, for which elasticity is not as important in the haptic touch, for instance parts of the anatomy models that are associated with muscles. Lighter foam layers may be suitable to reproduce fatty tissues which typically exhibit less hardness when pressed by a handheld instrument, while denser foams may rather replicate harder elements such as cartilage or hard cysts and growths that may be attached to the modeled organ, depending on the actual anatomy position and/or medical condition.

While various embodiments of an anatomy tissue model have been described above, it should be understood that they have been presented by way of example and not limitation. It will be apparent to persons skilled in the relevant art(s) that the proposed models and systems can be generalized to any type of anatomy tissue simulation applications, such as for instance veterinary applications, or adult entertainment. Various changes in form and detail can be made therein without departing from the spirit and scope. In fact, after reading the above description, it will be apparent to one skilled in the relevant art(s) how to implement alternative embodiments. Thus, the present embodiments should not be limited by any of the above-described embodiments.

In addition, it should be understood that any figures which highlight the functionality and advantages are presented for example purposes only. The disclosed methodology and system are each sufficiently flexible and configurable such that they may be utilized in ways other than that shown.

Although the term "at least one" may often be used in the specification, claims and drawings, the terms "a", "an", "the", "said", etc. also signify "at least one" or "the at least one" in the specification, claims and drawings.

## Claims

1. An anatomical model for simulating the interaction of a tool with a living body tissue, made of a fabric layer arranged over a foam layer to replicate the low-friction, elasticity and hardness of the living body tissue when touching the tissue with the tool, the fabric layer having a weight of at most 330g per square meter, the foam layer having a load deflection in the range of 1.5kPa to 19kPa and a weight in the range of 15kg to 110kg per cube meter.

2. The anatomical model of claim 1, wherein the fabric layer comprises at least one of PCM (phase change material), nylon or another synthetic polyamide, polyester, cotton, rayon, dyneema, Kevlar, Nomex, Lycra, spandex, acrylic fibres, or a combination thereof.

3. The anatomical model of claims 1 or 2, wherein the fabric layer comprises at least 5% of synthetic elastic fibre.

4. The anatomical model of claim 3, wherein the synthetic elastic fibre is elastane, Lycra, or Spandex.

5. The anatomical model of any of the claims 1 to 4, wherein the fabric layer has a weight of at least 80g per square meter.

6. The anatomical model of any of the claims 1 to 5, wherein the foam is a polyether foam.

7. The anatomical model of any of the claims 1 to 6, wherein the fabric layer and the foam layer are tethered by at least one tethering spot.

8. The anatomical model of claim 7, wherein the fabric layer and the foam layer are glued together.

9. The anatomical model of claim 7, wherein the fabric layer and the foam layer are screwed together.

10. A mannequin comprising at least one body structure and at least one anatomical model of any of the claims 1 to 9.

11. The mannequin of claim 10, wherein the body structure is made of plastic, silicon, rubber or polyurethane and at least one of the fabric layer or the foam layer of the anatomic tissue model system is tethered to the mannequin structure.

12. The mannequin of of claim 11, wherein at least one part of the fabric layer or at least one part of the foam layer of the anatomic model is glued or screwed to the mannequin structure.

13. An anatomical model insert for a mannequin, the mannequin comprising a cavity structure, the anatomical model insert being adapted to fast mount in the cavity structure, the anatomical model insert comprising at least one anatomical model of any of the claims 1 to 9.

14. The anatomical model insert of claim 13, further comprising an external structure arranged as an injection molded part, made of foam, plastic, or other suitable materials, to facilitate the insertion of the anatomical tissue model insert into the mannequin with a sliding fit.

15. The anatomical model insert of claims 13 or 14, wherein the fabric layer and/or the foam layer is firmly attached to the anatomy model external structure with various tethering techniques, such as glueing or screwing.
